(19) Europäisches Patentamt

European Patent Office

Office européen des brevets

(11) Publication number: **0 210 603 B1**

## (12) EUROPEAN PATENT SPECIFICATION

(45) Date of publication of patent specification: **18.12.91**

(51) Int. Cl.⁵: **G01N 33/38**, G01N 30/96, C11D 7/12, C11D 1/62, B28B 11/22, E04G 23/04, C11D 7/22

(21) Application number: **86110218.4**

(22) Date of filing: **24.07.86**

(54) Compositions for sulfation analysis, desulfation and cleaning of frescoed surfaces.

(30) Priority: **24.07.85 IT 2170685**

(43) Date of publication of application:
**04.02.87 Bulletin 87/06**

(45) Publication of the grant of the patent:
**18.12.91 Bulletin 91/51**

(84) Designated Contracting States:
**AT BE CH DE FR GB LI NL SE**

(56) References cited:
| | |
|---|---|
| **EP-A- 0 127 721** | **EP-A- 0 189 866** |
| **DE-A- 2 204 903** | **DE-A- 3 313 608** |
| **FR-A- 2 504 170** | **GB-A- 1 099 766** |

(73) Proprietor: **LARAC S.p.A.**
**13, via Sempione**
**I-21053 Castellanza (Varese)(IT)**

(72) Inventor: **Pizzigoni, Giuseppe**
**24, viale Stelvio**
**I-21052 Busto Arsizio Varese(IT)**
Inventor: **Parrini, Paolo**
**33, viale Volta**
**I-28100 Novara(IT)**

(74) Representative: **Zumstein, Fritz, Dr. et al**
**Dr. F. Zumstein Dr. E. Assmann Dipl.-Ing. F.**
**Klingseisen Bräuhausstrasse 4**
**W-8000 München 2(DE)**

## Description

The present invention concerns the use of a composition as defined below appropriate for the treatment of frescoed plaster coated surfaces with carbonate characteristics, to be used in particular for the measurement of the sulfation intensity present therein and/or for the removal of said sulfation as well as the protein material residues and finally for the removal, by selective action, of painted layers from the plastered surfaces when these last contain a chalky binder.

It is known that works of artistic interest, such as frescoes and wall decorations in general, undergo the action of aggressive elements contained in the atmosphere in amounts more and more dangerous: $SO_2$, $NO_x$, $CO_2$ and also that have been abandoned to the action of the elements owing to negligence or carelessness, with the subsequent insurgence of wall humidity, capillary transfer of soluble salts and fungus attacks.

It is known that one of the most dangerous types of deterioration for frescoed surface (by a fresco is meant a painting made with colors dispersed in water and applied to a surface plastered with lime putty and fine sand aggregate) is the one which is usually referred to as sulfation.

It can be brought about by impurities in the mortar and the aggregates employed, by wall component materials, by the action of salts transported by capillary humidity and by the combined action of condensation humidity with the sulfur dioxide contained in the air.

This unfortunately notorious phenomenon can be the reason of the total loss of works of noteworthy artistic value.

As a matter of fact, the transformation of calcium carbonate, which is the color binder in real frescoes, into dihydrate calcium sulphate (sulfation) causes a notable increase of the specific volume of the mass. This is followed by the creation of tensions and the growth of acicular or foliated crystals.

These phenomena, occuring in the painted layer, create liftings, scalings, separations and the formation of patinas, with the ensuing loss of adhesion.

In addition, if the sulfated painted surface were in contact with water, it would result in its irretrievable loss due to the considerable solubility of dihydrate calcium sulfate.

All activities directed to salvage and conserve pictorial works pertain to that difficult activity known simply as restoration.

Up to the time of introduction of synthetic materials one of the most used remedies for that purpose was to fight painted layer scalings with strengtheners based on more or less secret formulas, mostly of a protein type base: eggs and animal glues.

With aging these fresco strengtheners have almost always resulted in massive dimming phenomena, in the form of dark patinas and with the subsequent loss of the work color values (chromatism) and sign clarity.

Present trend is to intervene with restoration operations only after an exhaustive preliminary analytical survey, accompanied by scientific support activities. This allows the definition of the techniques that are most suited to the real requirements of the restoration process and that can be controlled both as to the final outcome as well as during the execution.

A classic operation for the removal of sulfation is the one that proposes to act on the same with ammonium carbonate solutions, so as not to jeopardize the work's integrity and so that a carbonate type surface is restored: the sulfuric ion is removed as ammonium sulfate by means of a double exchange reaction.

In EP-A-189 866 which is published on 6.8.86 there is described a composition containing a mixture of anion exchange material plus an aqueous solution of ammonium carbonate for the desulphatizing cleaning of carbonated stone surfaces.

To prevent other carbonated areas from becoming sulfatized again with the ammonium sulfate, a subsequent treatment is undertaken with barium hydroxide solutions, thus leading to the formation of a sulfate of low specific volume and highly inert.

In addition, the excess barium hydroxide can recarbonate and contribute to the plaster's reconsolidation.

Other compounds with a desulfation and cleaning action are known and also approved by some of the different boards or organizations operating in the protection of artistic and cultural properties areas. By way of example, there is the well known B57 whose action rests essentially upon the combined activity of ammonium carbonate, salts and detergent compounds in aqueous solution.

Said compounds are carefully applied and removed by sponging and other delicate mechanical operations after carefully controlled contact periods with the frescoed surfaces.

It is also known that in the chemical analysis preliminary operations, regularly undertaken before restoration takes place, the necessity arises for checking and eventually quantifying the frescoed surfaces sulfation intensity.

This can be done either qualitatively with contact reactions or quantitatively, after taking samples, which can never be large enough to represent the surfaces to be examined, in order to maintain the integrity of the work being studied.

The applicant has now discovered particular chemical compositions that applied to frescoed carbonate type plaster coated surface allow the following operations to be performed rapidly and easily without endangering the integrity of the painting:

- quantitative determination of surface sulfation level
- sulfation removal
- surface cleaning from residues of protein type strengthening materials

and lastly, when in presence of chalky type plaster, composed, by way of example, from plaster of Paris and marble powder:

- removal from the surface of said plaster through controlled disintegration of undesirable or deteriorated layers that might have been decorated; this is accomplished without damaging the underlying strata of precedent decorations that can thus be studied and restored.

These above mentioned compositions, object of this invention, consist of a mixture, generally a paste, of an ammonium carbonate aqueous solution with an anion exchange material in powder form.

This anion exchange material should preferably have its functional groups activated as, by way of example, in the form of amino groups or quaternary ammonium basis, not salified, with particle dimension preferably under 0.15 mm.

Examples of suitable anionic exchange materials are given by synthetic anionic exchange resins having basic functional groups, in particular resins with a matrix on a base of styrene polymers or copolymers of acrylic monomers or of polycondensation resins, or also from cross-linked and functionalized polysaccharides.

The preferred weight relationship between the exchange medium and the ammonium carbonate solution is of 0.80.

It has been shown that most suitable ammonium carbonate solutions of suitable concentration range from 10 to 15% by weight.

Such concentrations may be changed by subsequent approximations being led more by the extreme carefulness that must guide all interventions on art masterworks than by stoichiometric requirements.

Reduced granulometry of anion exchange media is useful when preparing humid mixtures with good spreading characteristics with the capacity of adhering well to the frescoed surfaces and of providing a mass with a thickness of at least 2 mm and of such microporosity that it can counteract the absorption of the solution through the plaster capillarity.

The mixture plaster action develops as long as it remains moist, and therefore, to counteract either the liquid absorption by capillarity as well as its drying out through evaporation, it is useful that an external protection be employed by applying a polythene sheet of the proper shape.

If the mixture is of the right consistency, it can be applied without difficulties to vertical surfaces as well as to ceilings.

In addition, it should remain active at least for two hours under normal temperature and relative humidity conditions as, by way of example, 15-20° C and 60-80% relative humidity.

After the application, the mixture has to be recovered quantitatively, dried, weighed, ground and homogenized before being used to perform the analyses required to determin its sulfur content.

RX fluorescence analysis, elementary microanalysis and colorimetric tests, can be usefully employed for testing.

The sulfur content as determined on the basis of the mixture recovered from the treatment, transformed in $SO''_4$ and related to the dimensions of the treated surface, gives an indication of sulfation intensity, for example in g $SO''_4$/sq.m.

Since the method shown is non-destructive, the tests can be repeated in different spots of the work being examined, allowing thus the acquisition of information that would not have been thought possible with techniques based on sample recovering.

Some useful changes can be made without jeopardizing the originality of the invention such as:

- granulometry and types of exchange materials variations;
- variations in the ratio between the anionic exchange materials and the ammonium carbonate solution;
- ammonium carbonate concentration variations;
- utilization with detergent agents, non-ionic surface-actives or cationic also with germicidal action.

Such changes can become necessary as a frescoed works being restored generally present problems that vary from point to point.

The paste action is developed by taking advantage of the calcium sulfate solubility in water: even if slight, it is not to be overlooked.

As a matter of fact, the continued elimination of sulfuric ions is possible as long as said paste remains humid.

This action is developed through their being fixed to the anionic exchange medium with the subsequent disintegration by solution of the new sulfate in the contact area.

Mixture dimensional shrinkage during the drying-out period causes the peeling-off of the topmost decorated layer in the area of contact with the plaster surface. It will adhere to the dry flakes and it will be possible to remove it with them.

For this application, too, the formulations given cannot be binding as the surface types to be treated can be very different, even within the same decoration.

All devices employed for controlling the following are very useful: mixture drying rate, its consistence, spreadability and stability on vertical surfaces and ceilings.

The following examples are given to illustrate the invention in more detail.

EXAMPLE 1

On two different areas of the deeply blackened surface of a 15th century fresco have been placed two small amounts of a mixture obtained by mixing the two components as indicated:

- 45 parts by weight: powdered exchange resin, strong anionic type, non salified (Kastel A500, Registered Trade Mark No. 132.799), particle size under 0.1 mm;
- 55 parts by weight: 15% ammonium carbonate solution.

The first position, 4.5 sq.cm, has been covered with a layer about 3 mm thick, while the second position, 3.5 sq cm, has been covered with a layer of equivalent thickness. This after the surfaces under study had been highly humidified by swabbing with a moist sponge.

The outer surfaces of the mixtures have been protected by covering them with small pieces of polyethylene film cut to the shape of the application.

After about 3.5 hours, the mixtures were believed to be dry and have been quantitatively removed to a calibrated weighing bottle, dried and weighed. They weighed respectively 0.325 and 0.334 g.

After grinding and homogenizing, they have been analyzed in RX fluorescence for their sulfur content, utilizing the $K_\alpha$ band intensity of the above named element and a specific calibration through microanalysis.

The sulfating levels of the two spots have been fixed as follows: 3.9 and 6.6 g $SO''_4$/sq.m., in perfect accord thus with destructive testing.

It turned out that the treated surfaces were also cleaned from black patina due to the residuates of protein strengthening materials.

Cleaning tests performed on other areas of the artwork by applying the compositions subject of this invention and modifying the contact time as a function of the painted layer have confirmed their effectiveness.

EXAMPLE 2

On a ceiling surface coated with a chalky plaster (plaster of Paris and marble powder) decorated by the superimposition of different colors ranging from yellow-brown up to dark-brown, a layer of about 2 mm thickness of an anionic exchange resin powder Kastel® A500, mixed with water in a weight ratio of about 45/55 has been spread with a small trawel.

After a drying period of about 2 hours appropriately slowed-down by the application of a polythene sheet, the flakes of dry mixture have been detached from the ceiling. It has been observed that the decoration was adhering to the surface that was in contact with the ceiling while on the plaster coating one could observe motifs of former decorations.

A second application in the same area has allowed the removal of the decorated plasters in same areas down to the last plaster coating on the wall.

The analysis of the exchange resins collected has confirmed fixing of the $SO''_4$ ions contained in an amount of 2.5-3.0%.

## Claims

1. Use of a desulphatizing composition consisting of a mixture of an ammonium carbonate aqueous solution and an anion exchange type powdered material for determining the degree of sulphation, desulphating and cleaning from residual protein type base material carbonate type plaster surfaces, characterized in that such surfaces are frescoed surfaces.

2. Use as claim 1 characterized in that the frescoed surfaces are frescoed plaster of Paris surfaces.

## Revendications

1. Application d'une composition de désulfatation, constituée d'un mélange d'une solution aqueuse de carbonate d'ammonium et d'un matériau en poudre de type échangeur d'anions, pour déterminer le degré de sulfatation, procéder à la désulfatation et débarrasser du matériau résiduel de base, de type protéine, les surfaces enduites d'un plâtre de type carbonate, caractérisée en ce que ces surfaces sont des surfaces peintes à fresque.

2. Application selon la revendication 1, caractérisée en ce que les surfaces peintes à fresque sont des surfaces de plâtre de Paris peintes à fresque.

## Patentansprüche

1. Verwendung einer Desulfatisierungszusammensetzung, bestehend aus einer Mischung einer wäßrigen Ammoniumcarbonatlösung und eines pulverförmigen Materials vom Anionenaustauschertyp zur Bestimmung des Sulfatierungsgrades, Desulfatierung und Reinigung von Carbonattyp-Gipsoberflächen von verbliebenem Basismaterial vom Proteintyp, dadurch gekennzeichnet, daß die Oberflächen mit Fresken versehene Oberflächen sind.

2. Verwendung gemäß Anspruch 1, dadurch gekennzeichnet, daß die mit Fresken versehenen Oberflächen mit Fresken versehene Kalkgips-Oberflächen sind.